# EUROPEAN PATENT APPLICATION

(11) **EP 4 049 671 A1**
(43) Date of publication of application: **31.08.2022**
(21) Application number: 22152608.0
(22) Date of filing: 06.07.2005
(51) Int. Cl.: A61K 38/00, A61K 9/08, A61L 27/22, A61L 27/54, A61K 47/42, C07K 1/00

(54) **PURIFIED AMPHIPHILIC PEPTIDE COMPOSITIONS AND USES THEREOF**

(30) Priority: 06.07.2004 US 585914 P
(62) Divisional of application: 05770153.4
(71) Applicant: 3D Matrix, Inc., Cambridge, MA 02139 (US)
(72) Inventor: Spirio, Lisa, Cambridge, 02139 (US); Chu, Zen, Brookline, 02445 (US)
(74) Representative: EIP

(57) **Abstract**

A method of preparing a composition comprising a plurality of amphiphilic peptide chains having alternating hydrophilic and hydrophobic amino acids, wherein the peptide contains at least 8 amino acids, are complementary and structurally compatible, and self-assemble into a beta-sheet macroscopic scaffold and wherein the method comprises a purification step. At least 75% of the peptide chains have the same sequence and the peptide chains are at least about 75% pure.

## Description

### Field of the Invention

The present invention relates to high purity amphiphilic peptide compositions.

### Background of the Invention

In general, the body is able to regenerate injured tissue to produce new tissue having properties similar to the original tissue. For example, small cuts heal without forming permanent scars, and clean fractures in bone are healed by the formation of new bone that binds the two fragments of bone together. However, connective tissue cells and other organ cells are anchorage dependent - they require a scaffold to exhibit normal physiological behavior. Where tissue damage is extensive or large gaps are present, cells migrating into the wound may not find proper anchorage and may produce scar tissue to bridge the gap between healthy tissue at the edges of the wound. Scar tissue does not have the same mechanical and biological properties as the original tissue. For example, scar tissue in skin is not as pliable as the original tissue. Scar tissue in bone is not as strong as uninjured bone and often provides a weak point where it is easier to break the bone again. Some tissues, such as articular cartilage, do not naturally regenerate, and healing only proceeds by the formation of scar tissue.

To date, there has been substantial effort expended to develop materials to replace or assist regeneration of various tissues. These materials sometimes exploit the ability of small wounds to heal by regeneration in tissues where large wounds heal by scar formation. Thus, materials are packed into a wound site that help bridge the gap between the edges of a wound and attempt to prevent the formation of scar before tissue regeneration proceeds. Although various synthetic and naturally derived materials have been developed for tissue regeneration, these materials sometimes suffer from immune incompatibility and improper distribution of stress. Furthermore, the use of material from animals, such as cow hide or cartilage from pigs or sharks, has raised concerns of possible contamination by infectious agents, such as prions. Thus, improved materials of biological origin that have improved compatibility, present a reduced risk of contamination, and provide the proper biomechanical characteristics for tissue repair are desirable.

### Definitions

By "scaffold" is meant a three-dimensional structure capable of supporting cells. Cells may be encapsulated by the scaffold or may be disposed in a layer on a surface of the scaffold. The beta-sheet secondary structure of the scaffold may be confirmed using standard circular dichroism to detect an absorbance minimum at approximately 218 nm and a maximum at approximately 195 nm. The scaffold is formed from the self-assembly of peptides that may include L-amino acids, D-amino acids, natural amino acids, non-natural amino acids, or a combination thereof. If L-amino acids are present in the scaffold, degradation of the scaffold produces amino acids which may be reused by the host tissue. It is also contemplated that the peptides may be covalently linked to a compound, such as a chemoattractant or a therapeutically active compound. The peptides may be chemically synthesized or purified from natural or recombinant sources, and the amino- and carboxy-termini of the peptides may be protected or not protected. The peptide scaffold may be formed from one or more distinct molecular species of peptides which are complementary and structurally compatible with each other. Peptides containing mismatched pairs, such as the repulsive pairing of two similarly charged residues from adjacent peptides, may also form scaffolds if the disruptive force is dominated by stabilizing interactions between the peptides. Scaffolds are also referred to herein as peptide structures, peptide hydrogel structures, peptide gel structures, or hydrogel structures.

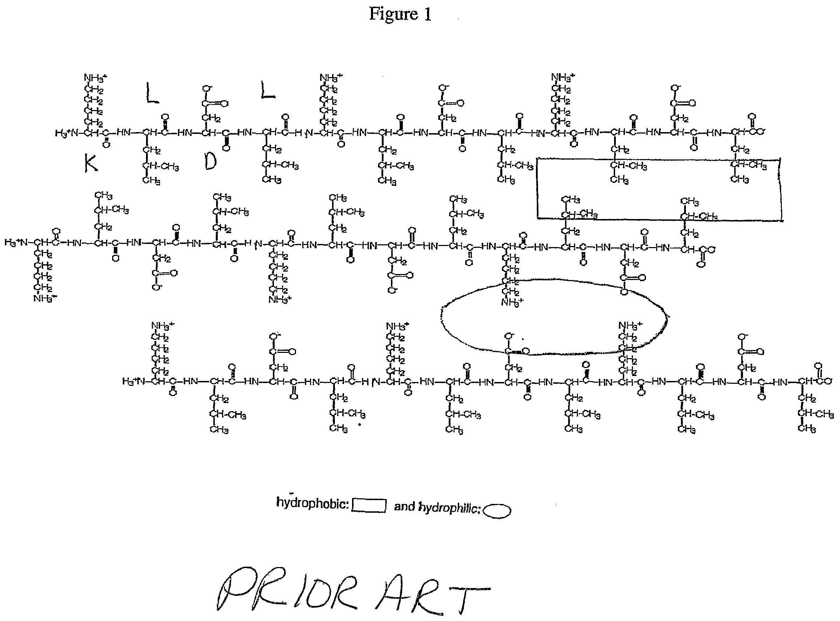

By "complementary" is meant capable of forming ionic or hydrogen bonding interactions between hydrophilic residues from adjacent peptides in the scaffold, as illustrated in Fig. 1, each hydrophilic residue in a peptide either hydrogen bonds or ionically pairs with a hydrophilic residue on an adjacent peptide or is exposed to solvent.

By "structurally compatible" is meant capable of maintaining a sufficiently constant intrapeptide distance to allow scaffold formation. In certain embodiments of the invention the variation in the intrapeptide distance is less than 4, 3, 2, or 1 angstroms. It is also contemplated that larger variations in the intrapeptide distance may not prevent scaffold formation if sufficient stabilizing forces are present. This distance may be calculated based on molecular modeling or based on a simplified procedure that has been previously reported (U.S. Patent Number 5,670,483). In this method, the intrapeptide distance is calculated by taking the sum of the number of unbranched atoms on the side-chains of each amino acid in a pair. For example, the intrapeptide distance for a lysine-glutamic acid ionic pair is 5+4=9 atoms, and the distance for a glutamine-glutamine hydrogen bonding pair is 4+4=8 atoms. Using a conversion factor of 3 angstroms per atom, the variation in the intrapeptide distance of peptides having lysine-glutamic acid pairs and glutamine-glutamine pairs *(e.g.,* 9 versus 8 atoms) is 3 angstroms.

The term "pure" is used to indicate the extent to which the peptides described herein are free of other chemical species, including deletion adducts of the peptide in question and peptides of differing lengths.

The term "biologically active agent" is used to refer to agents, compounds, or entities that alter, inhibit, activate, or otherwise affect biological or biochemical events. Such agents may be naturally derived or synthetic. Biologically active agents include classes of molecules (*e.g.*, proteins, amino acids, peptides, polynucleotides, nucleotides, carbohydrates, sugars, lipids, nucleoproteins, glycoproteins, lipoproteins, steroids, growth factors, chemoattractants, etc.) that are commonly found in cells and tissues, whether the molecules themselves are naturally-occurring or artificially created (*e.g.*, by synthetic or recombinant methods). Biologically active agents also include drugs, for example, anti-cancer substances, analgesics, and opioids. Preferably, though not necessarily, the drug is one that has already been deemed safe and effective for use by the appropriate governmental agency or body. For example, drugs for human use listed by the FDA under 21 C.F.R. §§330.5, 331 through 361, and 440 through 460; drugs for veterinary use listed by the DA under 21 C.F.R. §§500 through 589, incorporated herein by reference are all considered acceptable for use in accordance with the present invention. Additional exemplary biologically active agents include but are not limited to anti-AIDS substances, anti-cancer substances, immunosuppressants (*e.g.*, cyclosporine), anti-viral agents, enzyme inhibitors, neurotoxins, hypnotics, anti-histamines, lubricants, tranquilizers, anti-convulsants, muscle relaxants and anti-Parkinson agents, anti-spasmodics and muscle contractants including channel blockers, miotics and anti-cholinergics, anti-glaucoma compounds, anti-parasite, anti-protozoal, and/or anti-fungal compounds, modulators of cell-extracellular matrix interactions including cell growth inhibitors and anti-adhesion molecules, vasodilating agents, inhibitors of DNA, RNA or protein synthesis, antihypertensives, anti-pyretics, steroidal and non-steroidal anti-inflammatory agents, anti-angiogenic factors, anti-secretory factors, anticoagulants and/or antithrombotic agents, local anesthetics, ophthalmics, prostaglandins, targeting agents, neurotransmitters, proteins, cell response modifiers, and vaccines.

As used herein, a hydrogel such as a peptide hydrogel is "stable with respect to mechanical or physical agitation" if, when subjected to mechanical agitation, it substantially retains the physical properties (such as elasticity, viscosity, etc.), that characterized the hydrogel prior to physical agitation. The hydrogel need not maintain its shape or size and may fragment into smaller pieces when subjected to mechanical agitation while still being termed stable with respect to mechanical or physical agitation. The term "stable" does not have this meaning except when used with this phrase.

As used herein, the term "nanofiber" refers to a fiber having a diameter of nanoscale dimensions. Typically a nanoscale fiber has a diameter of 500 nm or less. According to certain embodiments of the invention a nanofiber has a diameter of less than 100 nm. According to certain other embodiments of the invention a nanofiber has a diameter of less than 50.nm. According to certain other embodiments of the invention a nanofiber has a diameter of less than 20 nm. According to certain other embodiments of the invention a nanofiber has a diameter of between 10 and 20 nm. According to certain other embodiments of the invention a nanofiber has a diameter of between 5 and 10 nm. According to certain other embodiments of the invention a nanofiber has a diameter of less than 5 nm.

The term "nanoscale environment scaffold" refers to a scaffold comprising nanofibers. According to certain embodiments of the invention at least 50% of the fibers comprising the scaffold are nanofibers. According to certain embodiments of the invention at least 75% of the fibers comprising the scaffold are nanofibers. According to certain embodiments of the invention at least 90% of the fibers comprising the scaffold are nanofibers. According to certain embodiments of the invention at least 95% of the fibers comprising the scaffold are nanofibers. According to certain embodiments of the invention at least 99% of the fibers comprising the scaffold are nanofibers. Of course the scaffold may also comprise non-fiber constituents, e.g., water, ions, growth and/or differentiation-inducing agents such as growth factors, therapeutic agents, or other compounds, which may be in solution in the scaffold and/or bound to the scaffold.

### Summary of the Invention

In one aspect, the invention is a composition including amphiphilic peptide chains having alternating hydrophilic and hydrophobic amino acids that are complementary and structurally compatible and self-assemble into a beta-sheet macroscopic scaffold. The peptide chains contain at least 8 amino acids, and at least about 75%, at least about 80%, at least about 85%, at least about 90%, at least about 95%, or at least about 99% of the peptide chains have the same sequence.

In another aspect, the invention is an aqueous solution comprising the peptide chains. The solution may form a hydrogel that is stable with respect to mechanical agitation. The solution may be injectable and may have a pH between about 4.5 and about 8.5. The peptides may be self-assembled into a matrix in the solution. The self-assembly need not be stable with respect to mechanical agitation. The solution may contain at least 0.1mM of an electrolyte.

The solution with the electrolyte may be injectable. The peptides may be adapted and constructed to be capable of self-assembly after injection. The concentration of the peptide chains in the aqueous solution may be at least about 1%, at least about 2% , at least about 3%, at least about 4%, at least about 5%, at least about 6%, at least about 7%, or at least about 8% by weight. Either the aqueous solution or the peptide chains may further include one or more biologically active agents.

In another aspect, the invention is a method of preparing a peptide chain. The method includes providing amino acids comprising a group in the side chain that is linked to a protecting group, assembling the amino acids into an amphiphilic peptide chain, removing the protecting group by reacting the peptide chain with an acid, and exchanging the acid for an non-fluorinated acetate salt. The non-fluorinated acetate salt may be selected from sodium acetate and potassium acetate. The acid may be trifluoroacetic acid.

In another aspect, the invention is a composition including an aqueous solution of greater than 2% amphiphilic peptide chains at least 8 amino acids long and having alternating hydrophilic and hydrophobic amino acids. The solution may but need not be mechanically stable with respect to self-assembly of the peptides.

In another aspect, the invention is a macroscopic scaffold comprising amphiphilic peptide chains, wherein the peptide chains have alternating hydrophobic and hydrophilic amino acids, are complementary and structurally compatible, and self-assemble into a beta-sheet macroscopic scaffold and wherein the amphiphilic peptide chains are in an aqueous solution, and wherein at least about 75% of the peptide chains have the same sequence. The macroscopic scaffold need not be mechanically stable with respect to physical agitation. The amphiphilic peptide chains may be in an aqueous solution containing an electrolyte, and, in this embodiment, the scaffold may be mechanically stable with respect to physical agitation. The peptide chains may be adapted and constructed to be capable of self-assembly after injection. A biologically active agent may be encapsulated within the scaffold.

In another aspect, the invention is a method of delivering a biologically active agent to a patient including providing an aqueous solution of amphiphilic peptides, adding a predetermined amount of the biologically active agent to the aqueous solution, and adding a predetermined amount of an electrolyte to the aqueous solution, wherein, after adding the predetermined amounts, the aqueous solution forms a hydrogel. The biologically active agent and the electrolyte may be combined in a single aqueous solution and added to the aqueous solution of the amphiphilic peptides simultaneously. The biologically active agent may be selected from an anti-inflammatory, an antibiotic, an anti-cancer agent, an analgesic, an opioid, a drug, a growth factor, a protein, an amino acid, a peptide, a polynucleotide, a nucleotide, a carbohydrate, a sugar, a lipid, a polysaccharide, a nucleoprotein, a glycoprotein, a lipoprotein, a steroid, a chemoattractant, and any combination of the above. The method may further include injecting the hydrogel into a predetermined site in a patient. For example, the method may include injecting the aqueous solution into a patient before adding a predetermined amount of electrolyte and after adding a predetermined amount of said biologically active agent, wherein adding a predetermined amount of electrolyte comprises allowing ions to migrate into the injected solution from surrounding tissue. In another example, the method may include injecting the aqueous solution into a predetermined site in a patient before adding a predetermined amount of electrolyte and after adding a predetermined amount of said biologically active agent, wherein the injected aqueous solution is stable with respect to migration from the predetermined site.

In another aspect, the invention provides a kit for delivering a peptide composition to a patient. The kit includes a purified peptide composition, which may be in the form of an aqueous solution, and at least one item selected from the group consisting of: an electrolyte, a buffer, a delivery device, a vessel suitable for mixing the peptide composition with one or more other agents. The delivery device may be, for example, a catheter, a needle, a syringe, or a combination of any of these. The kit may further include instructions for use, e.g., for preparing the peptide composition, for mixing the peptide composition with other agents, for introducing the peptide composition into a subject, etc.

In another aspect, the invention provides a kit for delivering a biologically active agent to a patient. The kit includes a purified peptide composition, which may be in the form of an aqueous solution comprising amphiphilic peptides, and the biologically active agent, which may be provided pre-mixed with the peptides or separately. The kit may further include an electrolyte, a buffer, a delivery device, instructions, etc. The biologically active agent may be present in nanospheres, microspheres, etc. (also referred to as nanocapsules, microcapsules, etc.). Numerous methods and reagents for making such spheres, capsules, etc., encapsulating a biologically active agent are known in the art. For example, standard polymers and methods for making sustained release and/or pH-resistant drug formulations can be used.

In another aspect, the invention provides a kit for delivering cells to a patient. The kit includes a purified peptide composition, which may be in the form of an aqueous solution comprising the peptides. The kit may further include cells. The kit may include any of the items listed in the description of the kits above. The kit may further include instructions for the preparation of cells to be delivered using the kit. For example, the instructions may describe how to culture cells, how to harvest cells from a subject, how to mix cells with the peptides, types of cells suitable for use, etc. In any of the inventive kits the aqueous solution may have a shelf life of at least nine weeks under predetermined conditions. The aqueous solution may further include an electrolyte.

### Brief Description of the Drawing

The invention is described with reference to the several figures of the drawing, in which,
**Figure 1** is a schematic illustration of the interactions between peptides in the peptide scaffold. Various peptides with amino acid sequences of alternating hydrophobic and hydrophilic residues self-assemble to form a stable scaffold of beta-sheets when exposed to physiologically-equivalent electrolyte solutions (U.S. Pat. Nos. 5,955,343 and 5,670,483). The peptide scaffolds are stabilized by numerous interactions between the peptides. For example, the positively charged and negatively charged amino acid side chains from adjacent peptides form complementary ionic pairs, and other hydrophilic residues such as asparagine and glutamine participate in hydrogen-bonding interactions. The hydrophobic groups on adjacent peptides participate in van der Waals interactions. The amino and carbonyl groups on the peptide backbone also participate in intermolecular hydrogen-bonding interactions.

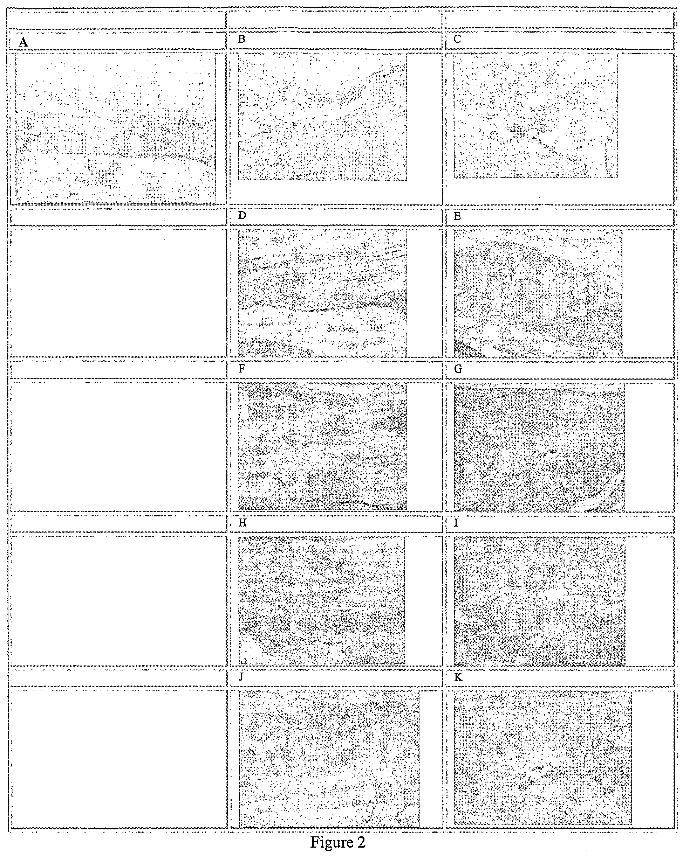

**Figure 2** is a series of photomicrographs depicting, for a rat calvarial defect 28 days after treatment, A) normal (uninjured) tissue, B) a control (untreated) calvarial defect, C) treatment with a 3% solution of unassembled peptide chains according to an embodiment of the invention, D) treatment with COLLAPLUG^{™}, E) treatment
with a 3% solution of peptide chains assembled in the presence of NaCl according to an embodiment of the invention, F) treatment with a 3% solution of peptide chains assembled in media according to an embodiment of the invention, G) treatment with a 3% solution of unassembled peptide chains combined with blood in a 1:1 ratio according to an embodiment of the invention, H) treatment with a combination of COLLAGRAFT^{™} and blood, I) treatment with a 3% solution of assembled peptide chains combined with tricalcium phosphate in a ratio of about 1:1 (volume of solution/weight of TCP) according to an embodiment of the invention, J) treatment with a combination of tricalcium phosphate and blood (1:1), and K) treatment with a 1% solution of assembled peptide chains in media according to an embodiment of the invention.

### Detailed Description of Certain Preferred Embodiments

The inventive compositions described herein include purified amphiphilic peptides having 12-16 amino acids. The peptides are at least 75% pure. In one embodiment, peptides may be prepared by providing amino acids including a group in the side chain that is linked to a protecting group, assembling the amino acids into an amphiphilic peptide, and removing the protecting group by reacting the peptide with an acetate salt in the absence of trifluoroacetic acid or any of its salts.

### Self-Assembling Peptides

Peptide sequences appropriate for use with the invention include those reported in U.S. Patents Nos. 5,670,483 and 5,955,343, and U.S. Patent Application No. 09/778,200, the contents of all of which are incorporated herein by reference. These peptide chains consist of alternating hydrophilic and hydrophobic amino acids that are capable of self-assembling to form an exceedingly stable beta-sheet macroscopic structure in the presence of electrolytes, such as monovalent cations. The peptide chains are complementary and structurally compatible. The side-chains of the peptide chains in the structure partition into two faces, a polar face with charged ionic side chains and a nonpolar face with alanines or other hydrophobic groups. These ionic side chains are self-complementary to one another in that the positively charged and negatively charged amino acid residues can form complementary ionic pairs. These peptide chains are therefore called ionic, self-complementary peptides, or Type I self-assembling peptides. If the ionic residues alternate with one positively and one negatively charged residue (-+-+-+-+), the peptide chains are described as "modulus I;" if the ionic residues alternate with two positively and two negatively charged residues (--++--++), the peptide chains are described as "modulus II." Exemplary peptide sequences for use with the invention include those listed in Table 1. In some embodiments, peptide sequences for use with the invention have at least 12 or 16 amino acid residues. Both D- and L- amino acids may be used to produce peptide chains. They may be mixed in the same chain, or peptide compositions may be prepared having mixtures of individual chains that themselves only include D- and L- amino acids.

**Table 1. Representative Self-Assembling Peptides**

| Name | Sequence (n-->c) | Modulus |
|---|---|---|
| RAD16-I | n-RADARADARADARADA-c | I |
| RGDA16-I | n-RADARGDARADARGDA-c | I |
| RADA8-I | n-RADARADA-c | I |
| RAD16-II | n-RARADADARARADADA-c | II |
| RAD8-II | n-RARADADA-c | II |
| EAKA16-I | n-AEAKAEAKAEAKAEAK-c | I |
| EAKA8-I | n-AEAKAEAK-c | I |
| RAEA16-I | n-RAEARAEARAEARAEA-c | I |
| RAEA8-I | n-RAEARAEA-c | I |
| KADA16-I | n-KADAKADAKADAKADA-c | I |
| KADA8-I | n-KADAKADA-c | I |
| KLD12 | n-KLDLKLDLKLDL-c | |
| EAH16-II | n-AEAEAHAHAEAEAHAH-c | II |
| EAH8-II | n-AEAEAHAH-c | II |
| EFK16-II | n-FEFEFKFKFEFEFKFK-c | II |
| EFK8-II | n-FEFKFEFK-c | I |
| KFE12 | n-FKFEFKFEFKFE-c | |
| KFE8 | n-FKFEFKFE-c | |
| KFE16 | n-FKFEFKFEFKFEFKFE-c | |
| KFQ12 | n-FKFQFKFQFKFQ-c | |
| KIE12 | n-IKIEIKIEIKIE-c | |
| KVE12 | n-VKVEVKVEVKVE | |
| ELK16-II | n-LELELKLKLELELKLK-c | II |
| ELK8-II | n-LELELKLK-c | II |
| EAK16-II | n-AEAEAKAKAEAEAKAK-c | II |
| EAK12 | n-AEAEAEAEAKAK-c | IV/II |
| EAK8-II | n-AEAEAKAK-c | II |
| KAE16-IV | n-KAKAKAKAEAEAEAEA-c | IV |
| EAK16-IV | n-AEAEAEAEAKAKAKAK-c | IV |
| RAD16-IV | n-RARARARADADADADA-c | IV |
| DAR16-IV | n-ADADADADARARARAR-c | IV |
| DAR16-IV^{∗} | n-DADADADARARARARA-c | IV |
| DAR32-IV | n-(ADADADADARARARAR)²-c | IV |
| EHK16 | n-HEHEHKHKHEHEHKHK-c | N/A |
| EHK8-I | n-HEHEHKHK-c | N/A |
| VE20^{∗} | n-VEVEVEVEVEVEVEVEVEVE-c | N/A |
| RF20^{∗} | n-RFRFRFRFRFRFRFRFRFRF-c | N/A |

| | | |
|---|---|---|
| N/A denotes not applicable ^{∗} These peptides form a β-sheet when incubated in a solution containing NaCl, however they have not been observed to self-assemble to form macroscopic scaffolds. | | |

Many modulus I and II self-complementary peptide sequences, such as EAK16, KAE16, RAD16, RAE16, and KAD16, have been analyzed previously (Table 1). Modulus IV ionic self-complementary peptide sequences containing 16 amino acids, such as EAK16-IV, KAE16-IV, DAR16-IV and RAD16-IV, have also been studied. If the charged residues in these self-assembling peptide chains are substituted (i.e., the positive charged lysines are replaced by positively charged arginines and the negatively charged glutamates are replaced by negatively charged aspartates), there are essentially no significant effects on the self-assembly process. However, if the positively charged resides, lysine and arganine are replaced by negatively charged residues, aspartate and glutamate, the peptide chains can no longer undergo self-assembly to form macroscopic scaffolds; however, they can still form a beta-sheet structure in the presence of salt. Other hydrophilic residues, such as asparagine and glutamine, that form hydrogen-bonds may be incorporated into the peptide chains instead of, or in addition to, charged residues. If the alanines in the peptide chains are changed to more hydrophobic residues, such as leucine, isoleucine, phenylalanine or tyrosine, these peptide chains have a greater tendency to self-assemble and form peptide matrices with enhanced strength. Some peptides that have similar compositions and lengths as the aforementioned peptide chains form alpha-helices and random-coils rather than beta-sheets and do not form macroscopic structures. Thus, in addition to self-complementarity, other factors are likely to be important for the formation of macroscopic scaffolds, such as the chain length, the degree of intermolecular interaction, and the ability to form staggered arrays.

Other self-assembling peptide chains may be generated by changing the amino acid sequence of any self-assembling peptide chains by a single amino acid residue or by multiple amino acid residues. Additionally, the incorporation of specific cell recognition ligands, such as RGD or RAD, into the peptide scaffold may promote the proliferation of the encapsulated cells. *In vivo,* these ligands may also attract cells from outside a scaffold to the scaffold, where they may invade the scaffold or otherwise interact with the encapsulated cells. To increase the mechanical strength of the resulting scaffolds, cysteines may be incorporated into the peptide chains to allow the formation of disulfide bonds, or residues with aromatic rings may be incorporated and cross-linked by exposure to UV light. The *in vivo* half-life of the scaffolds may also be modulated by the incorporation of protease cleavage sites into the scaffold, allowing the scaffold to be enzymatically degraded. Combinations of any of the above alterations may also be made to the same peptide scaffold.

Self-assembled nanoscale structures can be formed with varying degrees of stiffness or elasticity. While not wishing to be bound by any theory, low elasticity may be an important factor in allowing cells to migrate into the scaffold and to communicate with one another once resident in the scaffold. The peptide scaffolds described herein typically have a low elastic modulus, in the range of 1-10 kPa as measured in a standard cone-plate rheometer. Such low values permit scaffold deformation as a result of cell contraction, and this deformation may provide the means for cell-cell communication. In addition, such moduli allow the scaffold to transmit physiological stresses to cells migrating therein, stimulating the cells to produce tissue that is closer in microstructure to native tissue than scar. Scaffold stiffness can be controlled by a variety of means including changes in peptide sequence, changes in peptide concentration, and changes in peptide length. Other methods for increasing stiffness can also be used, such as by attaching a biotin molecule to the amino- or carboxy-terminus of the peptide chains or between the amino-and carboxy- termini, which may then be cross-linked.

Peptide chains capable of being cross-linked may be synthesized using standard f-moc chemistry and purified using high pressure liquid chromatography (Table 2). The formation of a peptide scaffold may be initiated by the addition of electrolytes as described herein. The hydrophobic residues with aromatic side chains may be cross-linked by exposure to UV irradiation. The extent of the cross-linking may be precisely controlled by the predetermined length of exposure to UV light and the predetermined peptide chain concentration. The extent of cross-linking may be determined by light scattering, gel filtration, or scanning electron microscopy using standard methods. Furthermore, the extent of cross-linking may also be examined by HPLC or mass spectrometry analysis of the scaffold after digestion with a protease, such as matrix metalloproteases. The material strength of the scaffold may be determined before and after cross-linking, as described herein.

**TABLE 2 Representative Peptide Sequences for Cross-Linking**

| Name | Sequence (N-->C) |
|---|---|
| RGDY16 | RGDYRYDYRYDYRGDY |
| RGDF16 | RGDFRFDFRFDFRGDF |
| RGDW16 | RGDWRWDWRWDWRGDW |
| RADY16 | RADYRYEYRYEYRADY |
| RADF16 | RADFRFDFRFDFRADF |
| RADW16 | RADWRWDWRWDWRADW |

Aggrecan processing sites, such as those underlined in Table 3, may optionally be added to the amino- or carboxy-terminus of the peptides or between the amino- and carboxy-termini. Likewise, other matrix metalloprotease (MMP) cleavage sites, such as those for collagenases, may be introduced in the same manner. Peptide scaffolds formed from these peptide chains, alone or in combination with peptides capable of being cross-linked, may be exposed to various proteases for various lengths of time and at various protease and peptide concentrations. The rate of degradation of the scaffolds may be determined by HPLC, mass spectrometry, or NMR analysis of the digested peptide chains released into the supernatant at various time points. Alternatively, if radiolabeled peptide chains are used for scaffold formation, the amount of radiolabeled material released into the supernatant may be measured by scintillation counting. For some embodiments, the beta-sheet structure of the assembled peptide chains is degraded sufficiently rapidly that it is not necessary to incorporate cleavage sites in the peptide chains.

**TABLE 3 Representative Peptide Sequences having Aggrecan Processing Sites**

| Name | Sequence (N-->C) |
|---|---|
| REEE | RGDYRYDYTFREEE-GLGSRYDYRGDY |
| KEEE | RGDYRYDYTFKEEE-GLGSRYDYRGDY |
| SELE | RGDYRYDYTASELE-GRGTRYDYRGDY |
| TAQE | RGDYRYDYAPTAQE-AGEGPRYDY-RGDY |
| ISQE | RGDYRYDYPTISQE-LGQRPRYDYRGDY |
| VSQE | RGDYRYDYPTVSQE-LGQRPRYDYRGDY |

If desired, peptide scaffolds may also be formed with a predetermined shape or volume. To form a scaffold with a desired geometry or dimension, an aqueous peptide solution is added to a pre-shaped casting mold, and the peptide chains are induced to self-assemble into a scaffold by the addition of an electrolyte, as described herein. The resulting geometry and dimensions of the macroscopic peptide scaffold are governed by the concentration and amount of peptide solution that is applied, the concentration of electrolyte used to induce assembly of the scaffold, and the dimensions of the casting apparatus.

If desired, peptide scaffolds may be characterized using various biophysical and optical instrumentation, such as circular dichroism (CD), dynamic light scattering, Fourier transform infrared (FTIR), atomic force microscopy (ATM), scanning electron microscopy (SEM), and transmission electron microscopy (TEM). For example, biophysical methods may be used to determine the degree of beta-sheet secondary structure in the peptide scaffold. Additionally, filament and pore size, fiber diameter, length, elasticity, and volume fraction may be determined using quantitative image analysis of scanning and transmission electron microscopy. The scaffolds may also be examined using several standard mechanical testing techniques to measure the extent of swelling, the effect of pH and electrolyte concentration on scaffold formation, the level of hydration under various conditions, and the tensile strength.

### Production of Peptides

Peptide chains for use with the invention may be produced using techniques well known to those skilled in the art, including solution phase synthesis and solid phase synthesis. These techniques may be optimized for production of the peptide chains described herein at purity levels that provide the resulting composition with surprising properties, including but not limited to shelf life, degradation rate, solubility, and mechanical characteristics.

In one embodiment, peptide chains for use with the invention are produced using solid phase peptide synthesis techniques. The synthesis may be carried out at room temperature in a glass reactor vessel, for example, with a coarsely porous glass-fritted disk of coarse porosity in the bottom. The reactor facilitates the addition of amino acid derivatives, solvents, and reagents used in the reaction. One skilled in the art will recognize that the size of the reactor depends on the amount of resin used for the synthesis. The reactor vessel may be equipped with a mechanical stirrer or placed on a platform shaker to effect efficient mixing of the peptide-resin complex with the reaction solution.

One skilled in the art will be familiar with a variety of resins that may be used to support peptide chains as they are being synthesized. An exemplary resin for use with the techniques of the invention is Rink Amide MBHA Resin, available from Glycopep Chemicals, Inc. (Chicago, IL). MBHA resin is a 1% divinylbenzene (DVB) cross-linked polystyrene resin derivatized with 0.4-0.8 mmole/gram of 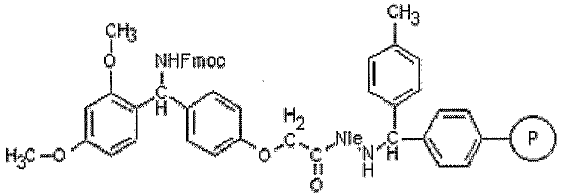 where Fmoc is N-alpha-(9-fluorenylmethyloxycarbonyl) and Nle is norleucine, which is used as a marker to determine the level of substitution in the resin. While the substitution level of the polymer does not significantly affect product quality, it significantly affects manufacturing times and costs. Too high a substitution level may result in prolonged coupling times, while too low a substitution level increases the quantity of resin required to perform the reaction and the volume of solvents required for the washing steps. Additional resins include but are not limited to chloromethylpolystyrene (CMS) resin, 4-hydroxyphenoxymethyl polystyrene resin, Risk Amide AMS resin 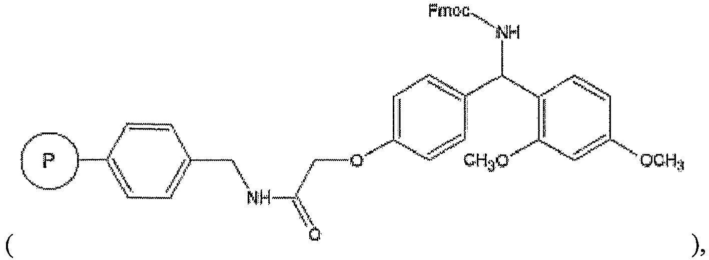 and sarcosine dimethyl acrylamide resin 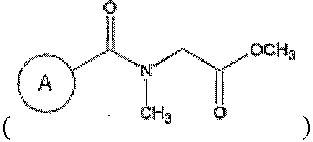 all available from Polymer Laboratories. These resins may be purchased with a particular amino acid already attached to the resin.

Where peptide chains are synthesized from the C-terminal amino acid, both the reactive side chain and the alpha amino group of the amino acid being added to the peptide chain should be protected. A labile protecting group such as Fmoc may be used to block the alpha amino group, while a stable protecting group may be used to block reactive side chains. In one embodiment, the labile protecting group is removed by piperidine, and the stable protecting group is removed by strong acid, as described below. Exemplary protecting groups for amino acid side chains include but are not limited to 2,2,4,6,7-pentamethyldihydrobenzofuran (Pbf), tert-butoxy (OtBu), trityl (Trt), tert-butyl (tBu), and Boc (tert-butoxycarbonyl). One skilled in the art will recognize which protecting groups are appropriate for specific amino acids. Amino acids with protecting groups already in place are available commercially, for example, from Advanced ChemTech, Louisville, KY.

To add an amino acid to a growing peptide chain, the alpha amino group at the end of the resin terminated chain is acylated by the next activated amino acid being added to the peptide chain. In one embodiment, the protected amino acid and 2-(1H-benzotriazole -1-yl)-1,1,3,3-tetramethyluroniunihexafluorophosphate (HBTU) are dissolved in DMF. N-methylmorpholine (NMM) is added to the solution, which is then added to the resin. In general, the reagents for acylation are selected to optimize the reaction conditions and for ease of elimination after coupling.

The HBTU/NMM/resin mixture is allowed to react for an appropriate interval, for example, at least one hour, after which the extent of reaction may be determined using the TNBS test (Hancock, et al., Anal. Biochem., 1976, 71, 260) and/or Ninhydrin test (e.g., reaction of sample with ninhydrin, amines identified colorimetrically). If residual amino groups are detected, the coupling reaction may be repeated using half the amount of reagent required for the initial coupling reaction. Unreacted alpha amino acids still present after repetition of the reaction may be capped with acetic anhydride to avoid deletion sequences in following cycles.

After each amino acid is added, the labile Fmoc protecting group may be cleaved from the alpha amino function of the N-terminal amino acid on the growing peptide by treating the resin twice with a 20% solution of piperidine in DMF or a DMF mixture with water. In this embodiment, the stable protecting group is resistant to removal under these conditions and remains attached to the amino acid.

After both the coupling reaction and the deprotection reaction, the resin may be washed with DMF to eliminate excess reagent. DMF is an excellent solvent for the reagents used in the coupling step and also has excellent swelling properties. Alternative solvents may also be used to produce peptide chains for use with the invention. Such solvents should be selected to minimize the risk of side reactions while efficiently extracting excess reagent from the reaction mixture. Rinse times should be sufficient to allow for thorough contact of the peptide-resin with the solvent and for extraction of the reagents. One skilled in the art will recognize that the washing steps may be repeated but that repeated washing will increase the cost of manufacture.

After the last amino acid in the sequence has been added, the N-terminal of the peptide may be capped with acetic anhydride and the completed chain detached from the resin. Any side-chain protecting groups are then cleaved. This may be accomplished by treatment of the peptide-resin complex with trifluoroacetic acid in the presence of scavengers, for example, water, phenol, and/or triisopropylsilane. Scavengers trap reactive cations during cleavage and prevent alkylation of reactive side chains.

In an alternative embodiment, the peptide chains are synthesized using solution phase techniques. Solution phase synthesis is based on stepwise addition of protected amino acids to the growing peptide chain. This method is faster than solid phase methods for peptide chains having a repeating unit, such as RAD16. For example, the four-mer RADA may be produced in several reaction vessels. In one of the reaction vessels, the C-terminal amino acid is deprotected and the N-terminal acid is protected. Addition of the C-terminal-unprotected four-mer to a second reaction vessel doubles the length of the peptide in a single reaction step. The process is repeated to produce a 12-mer. One skilled in the art will recognize that several combinations of four-mers with one another will result in rapid production of the 16-mer.

The completed peptides may be purified using standard peptide purification techniques, including gel filtration and ion exchange high pressure liquid chromatography (HPLC). In one embodiment, the peptide chains are purified using reverse phase HPLC with PLRP-S, a DVB-crosslinked polystyrene available from Polymer Laboratories, Inc., Amherst, MA. The separation is based on hydrophobic interactions between the peptide and the polymer. The interaction of the peptide chains with the resin is sufficiently specific that peptide chains of similar structures may be readily separated using the resin support. In general, the column is washed with aqueous acetic acid to equilibrate it. Crude peptide is loaded onto the column and eluted using a gradient of acetic acid/water and acetonitrile/acetic acid/water. In one embodiment, 0.1% acetic acid and 80% acetonitrile are used, but one skilled in the art will recognize that the proportions may be adjusted to optimize processing times and the separation between the desired peptide and various impurities. Likewise, the gradient may be a 0.5%-1.0% (e.g., increase in acetonitrile solution) change per minute but may be adjusted to optimize the separation between the reaction product and impurities. The purification method also exchanges acetate for trifluoroacetate, converting the peptide to the acetate salt form. The acetate-exchanged product is more biocompatible than the fluorinated product. Unexpectedly, use of sodium acetate also results in a more highly purified product with fewer deletion adducts.

As fractions are collected, the content of the fraction may be monitored by analytical reverse phase HPLC eluted with 0.1%TFA in aqueous acetonitrile. Any reverse phase HPLC support that could be used to purify the peptide product may also be used to analyze the quality of the eluted fractions. In one embodiment, a C18 column (e.g, using silica derivatized with C18 chains) may be used. Fractions from the purification column that meet the desired purity specification may be pooled and lyophilized. Fractions with lesser purity may be reprocessed. Fractions that cannot be reprocessed to obtain higher purity material may be discarded.

The purification step separates peptide chains having the desired sequence from several types of byproducts. The first is excess reagents, from which the desired peptide sequence is easily separated because it has a different chemical structure and higher molecular weight. The second includes deletion adducts, peptide chains similar in sequence to the desired peptide but which have one or more missing amino acids. We have unexpectedly found that enhanced removal of deletion adducts increases the solubility of the desired peptide chains in aqueous media and changes various properties of the self-assembled scaffolds. In some embodiments, the final product may be at least 75%, at least 80%, at least 85%, at least 90%, at least 95%, or at least 99% pure. In some embodiments at least 75%, at least 80%, at least 85%, at least 90%, at least 95%, or at least 99% of the individual peptide chains in a final peptide product are identical in length and sequence.

### Preparation of Peptide Solution and its Characteristics

The purified product may be stored as a powder or may be redissolved in aqueous solution. The peptide product is significantly more soluble in water than previous formulations, and concentrations greater than 2% or 3% may be achieved. Agitation, for example, using sonication or a shaker table, helps the peptide chains go into solution. The concentration of peptide chains in solution may be varied depending on the desired application. In one embodiment, the concentration of peptide chains in water may be between about 0.25% and about 7%, for example, about 0.5%, about 1%, about 2%, about 3%, about 4%, about 5%, or about 6% (all concentrations are in weight percent unless otherwise indicated). In another embodiment, the concentration of peptide chains may be even greater, for example, between about 7% and about 10%, for example, about 8% or about 9%.

The peptide chains in solution spontaneously self-assemble into scaffolds through electrostatic interactions. The self-assembled peptide chains form a hydrogel that remains ductile and amenable to flow upon application of an appropriate stimulus. In some embodiments, the self-assembly may be interrupted by physically agitating the hydrogel/solution, for example, by vortexing, sonicating, or simply administering a sharp tap to the container holding the gel.

The peptide solution may have a shelf life of at least one year with or without added electrolyte (see below).

The peptide solution may be radiation sterilized, if desired. In one embodiment, the peptide solution is stable with respect to exposure to gamma radiation to about 35 Kgrey. Less radiation, e.g., about 25 Kgrey, may be used for sterilization if it will be sufficient. Peptide solutions produced using the techniques described herein are stable with respect to exposure to radiation and do not experience structural alterations upon exposure to sterilizing radiation. Of course, peptide solutions may also be sterilized by injection through a 0.45 micron filter.

### Formation of a Self-Assembled. Peptide Scaffold and its Properties

The peptide solutions may be formed into a stable scaffold by exposure to a monovalent salt solution. Sufficient electrolyte is added to the solution to initiate self-assembly of the peptides into a beta-sheet macroscopic structure. In certain embodiments of the invention, the concentration of the added electrolyte is at least 5, 10, 20, or 50 mM. Smaller concentrations, e.g., 0.1 to 1 mm, or larger concentrations may also be used. The choice of concentration depends partially on the desired ionic strength of the peptide gel and also affects the speed of gelation. Suitable electrolytes include, but are not limited to, Li⁺, Na⁺, K⁺, and Cs⁺. The electrolyte causes the peptide chains to self-assemble into a scaffold that is stable with respect to mechanical agitation.

At low concentrations, addition of electrolyte to the peptide solution results in an agar-like gel that exhibits limited liquid flow and high flexibility. The mechanical behavior of low peptide concentration hydrogels is similar to that of gelatin. While highly flexible, the hydrogel is also brittle. Rather than plastically deforming, the gel breaks into separate pieces or fractures. We have found that these properties are not duplicated at higher concentrations. Rather, higher concentration gels remain ductile and coherent and are amenable to plastic deformation. With a consistency resembling that of long chain hyaluronic acid, they may be injected through a 30 gauge needle.

At higher concentrations, solutions of peptide chains behave as non-Newtonian fluids. In addition, the solution becomes more viscous over time. For example, a 3% solution exhibited a yield stress of 20-30 Pa and a viscosity less than 40 cP about an hour after mixing, with the yield stress increasing to 50-65 Pa after 5-6 hours. After two weeks, the yield stress increased to 100-160 Pa, and the viscosity increased to less than 200 cP.

The ductile gel behaves as a injection-molded material. Peptide hydrogels passed through a needle fill a desired space with a single coherent bolus rather than a tangled, threaded mass. That is, the material assembles both at the scale of the individual peptide chains and on a macroscale as a gel. It provides both an injectable material for ease of administration and a continuous fibrous network that facilitates cellular ingrowth and proliferation.

We have also discovered that high purity (*e.g.*, with respect to chain composition) peptide gels exhibit accelerated degradation in comparison to lower purity gels. Indeed, gels produced using the techniques of the invention exhibit faster degradation *in vivo* than collagen or hyaluronic acid. For example, a bolus of 200µl of 1% RAD16 produced using the techniques of the invention and implanted *in vivo* was degraded and excreted within 28 days. Over 90 % of a 70µl bolus of 1% RAD16 was degraded and excreted within 14 days. Higher concentration gels exhibit longer degradation times.

In certain embodiments of the invention high purity gels are also stable at physiological pH. Gels may be brought to physiological pH by equilibrating them with a buffer solution. For example, a layer of buffered solution having the desired pH may be charged above or below a layer of a peptide gel produced using the techniques of the invention. This approach has been used, for example, to equilibrate peptide scaffolds with phosphate buffered saline or tissue culture medium prior to use of the scaffolds for culturing cells. After the gel and the buffer solution have equilibrated for some amount of time, the pH of the gel is easily tested by removing the solution and putting a strip of pH paper in or on the gel. One skilled in the art will recognize that it may be necessary to repeat the process several times to bring the gel to the desired pH. Alternatively or in addition, an excess of buffer may be used. A rocker may also be used to speed equilibration of the buffer and the gel. Gels at physiological pH may retain the physical properties that they had at lower pH. They may still be injected or combined with cells. Of course, such gels are much more compatible with physiological tissue and cells at physiological pH than at pH 2-3.

According to certain embodiments of the invention it is desirable to modify the pH of a peptide solution, e.g., to raise the pH to a physiological pH of ~7 - 8.5 prior to introducing the peptide solution into a subject (see below) or prior to encapsulating cells. The pH-modified peptide solutions may be used for other purposes also, e.g., for tissue culture or for encapsulating biologically active agents. It may be desirable to achieve the pH modification in a relatively rapid manner, e.g., without the need for prolonged equilibration with buffer solution and/or multiple changes of buffer solution.

A number of different buffers were tested in order to assess their ability to raise the pH of a 1% aqueous solution of peptide. RAD16-I peptide was used for this study, and the following buffers were tested:
1. ammonium acetate (laboratory prepared)
2. sodium citrate (laboratory prepared)
3. sodium acetate (laboratory prepared)
4. sodium bicarbonate (clinical grade made by Abbott, Inc.),
5. HEPES (research grade made by Stem Cell Technologies, Inc.)
6. Tris-HCl (laboratory prepared)
7. Tromethamine, also termed THAM (clinical grade made by Abbott, Inc.).
8. Phosphate buffered saline without Ca or Mg (Gibco-BRL)

All these buffers were able to increase the pH of the peptide solution without destroying the ability of the peptide chains to assemble into a scaffold, either before or after the addition of an electrolyte. Assembled scaffolds can also be equilibrated with a buffer to raise the pH. Tables 4 and 5 summarize the results obtained in these experiments. In the tables, "Pre-Salt" refers to experiments in which an electrolyte was added to the peptide composition prior to addition of the buffer solution, and "Post-Salt" refers to experiments in which an electrolyte was added to the peptide composition following addition of the buffer. The "Pre-Salt" and "Post-Salt" columns indicate the final buffer concentration and the pH of the peptide solution. The buffers listed enable the peptide gel to achieve pH's between 4.5 and 8.5. The resulting peptide gels were not stable with respect to mechanical agitation. For example, when buffer was stirred into an assembled gel, the peptide structure disassembled and became runny and watery. A similar phenomenon was observed when electrolyte was stirred into unbuffered solutions. Such pH values are compatible with *in vivo* uses of the peptide solutions and hydrogels, as are various in between pH values, e.g., 6.0, 6.5, 7.0, 7.5, 8.0, etc.

In both sets of experiments, the pH of peptide solutions and gels was determined by contacting the peptide solution or assembled peptide scaffold with pH paper that had been calibrated against standard pH calibration solutions. The peptide solution was either removed from a vessel containing it and squirted onto pH paper using a pipette or, in the case of peptide gels that remained assembled in the presence of the buffer solution, the buffer solution was first taken off and the peptide gel was then removed from the vessel using a spatula and placed in contact with the pH paper.

It is noted that the experimental data reported herein is provided for exemplary purposes and is not intended to limit the invention. Other buffers, other buffer concentrations, etc., are within the scope of the invention.

**Table 4: Results-Mixing buffer into 1% peptide composition:**

| Buffer | Pre-Salt (NaCl added to 1% at 0.9% then buffer added) | Post-Salt (buffer added to 1% then NaCl add to 0.9%) | No salt (buffer only) |
|---|---|---|---|
| 1. ammonium acetate, 1.0 M, pH 5.5 (lab prepared) | 10 mM, pH 5.0 | 10 mM, pH 5.0 | 10 mM, pH 5.0 |
| 2. sodium citrate, 1.0 M, pH 6.5) (lab prepared) | 10 mM, pH 5.5 | 10 mM, pH 5.5 | 10 mM, pH 5.5 |
| 3. sodium acetate, 3.0 M, pH 5.5 (lab prepared) | 10 mM, pH 5.0 | 10 mM, pH 5.0 | 10 mM, pH 5.0 |
| 4. sodium bicarbonate, 8.4%, pH 7.8 (clinical grade, Abbott, Inc.) | 0.084%, pH 4.5 | 0.084%, pH 4.5 | 0.084%, pH 4.5 |
| 5. HEPES, 1.0 M, pH 7.2 (research grade, Stem Cell Technologies, Inc.) | 10 mM, pH 4.5 | 10 mM, pH 4.5 | 10 mM, pH 4.5 |
| 6. Tris-HCl, 1.0 M, pH 8.5 (lab prepared) | 10 mM, pH 5.5 | 10 mM, pH 5.5 | 10 mM, pH 5.5 |
| 7. Tromethamine, termed THAM, 0.3 M, pH 8.6 (clinical grade, Abbott, Inc.). | 10 mM, pH 5.5 | 10 mM, pH 5.5 | 10 mM, pH 5.5 |
| 8. no buffer (1% PuraMatrix in water) | pH 3.0 | pH 3.0 | pH 2.5 |

**Table 5: Results-Overlay of buffer on top of peptide composition:**

| Buffer | Pre-Salt (NaCl added to 1% at 0.9% then buffer added) | Post-Salt (buffer added to 1% then NaCl added to 0.9%) | No salt (buffer only) |
|---|---|---|---|
| 1. ammonium acetate, 1.0 M, pH 5.5 (lab prepared) | 10 mM, pH 5.0 | 10 mM, pH 5.0 | 10 mM, pH 5.0 |
| 2. sodium citrate, 1.0 M, pH 6.5) (lab prepared) | 10 mM, pH 5.5 | 10 mM, pH 5.5 | 10 mM, pH 5.5 |
| 3. sodium acetate, 3.0 M, pH 5.5 (lab prepared) | 10 mM, pH 5.0 | 10 mM, pH 5.0 | 10 mM, pH 5.0 |
| 4. sodium bicarbonate, 8.4%, pH 7.8 (clinical grade, Abbott, Inc.) | 8.4%, pH 7.8 | 8.4%, pH 7.8 | 8.4%, pH 7.8 |
| 5. HEPES, 1.0 M, pH 7.2 (research grade, Stem Cell Technologies, Inc.) | 10 mM, pH 4.5 | 10 mM, pH 4.5 | 10 mM, pH 4.5 |
| 6. Tris-HCl, 1.0 M, pH 8.5 (lab prepared) | 10 mM, pH 5.5 | 10 mM, pH 5.5 | 10 mM, pH 5.5 |
| 7. Tromethamine, termed THAM, 0.3 M, pH 8.6 (clinical grade, Abbott, Inc.). | 0.3 M, pH 8.5 | 0.3 M, pH 8.5 | 0.3 M, pH 8.5 |
| 8. PBS, no Ca or Mg, pH 7.4 (Gibco-BRL) | pH 7.4 | pH 7.4 | pH 7.4 |
| 9. no buffer (1% PuraMatrix in water) | pH 3.0 | pH 3.0 | pH 2.5 |

### Use of Self-Assembled Peptide Scaffolds as Tissue Fillers

The peptide gels described herein provide a matrix to which cells may attach and on which they may migrate into the interior of a wound site. The peptide scaffolds disclosed herein comprise a network of nanofibers with intervening spaces rather than a solid matrix. Such a structure may allow cell infiltration and cell-cell interaction in a way that more closely resembles the setting of cells within the body than that allowed by other culture techniques and materials. Instead of merely healing from the edges in, the entire area of the wound may be regenerated concurrently as cells migrate to the center of the scaffold.

High purity gels according to an embodiment of the invention may be directly injected into wound sites to facilitate wound healing. For example, they may be injected into biopsy sites or wound sites created by the removal of a tumor. The gels may also be used to facilitate healing in chronic wounds, such as skin lesions and diabetic ulcers. The use of peptide hydrogels to facilitate healing in nervous tissue is discussed in U.S. Application No. 10/968,790. Of course, the peptide gels may be used to promote tissue regeneration in non-surgically created wound sites. Because the gels may be injected, there is no need to enlarge a wound site beyond what was originally needed to remove diseased tissue. Furthermore, the gel does not need to be shaped to fit the wound site. Rather, it simply fills the wound site the way a liquid fills a container into which it is poured. Because the injected gel forms a coherent bolus rather than individual strands or threads, it easily penetrates nooks and crevices at the edges of a rough wound.

Alternatively or in addition, the peptide gels may be used as bulking agents. For example, peptide gels or solutions may be injected under the skin to fill in tissue depressions resulting from scars. They may also be used in place of collagen or hyaluronic acid injections to fill out sagging skin and fill in wrinkles. Large dimples may also result from large subcutaneous wounds, for example, severe injuries to the skull or mandible. Peptide gels may be used to fill out such dimples whether or not it is desired to have the gel remodel into natural tissue or a fibrous tissue capsule. For example, peptide gels may be used for breast augmentation. In this embodiment, it is not necessary that the gel be remodeled into mammary tissue. In another embodiment, gels may be injected subcutaneously to cause the skin to stretch. For example, when a flap of skin is needed for surgery, the skin may be produced autologously by injecting a bolus of gel under the skin near the surgical site or elsewhere on the body. The pressure from the bolus of gel stretches the existing skin. To relieve the pressure, the body produces more skin in the area of the bolus, much as the body produces new skin to accommodate a pregnancy. Additional gel may be added over time to increase the size of the bolus.

Internal tissues may also be augmented with peptide gels. For example, gels may be injected into the urethra to prevent reflux or correct incontinence. In a related application, the gels described herein may be used for embolization. For example, gels may be injected into blood vessels around a tumor or vessels that have been cut during surgery to stop blood flow. The use of peptide gels as hemostatic agents is discussed in U.S. Provisional Application No. 60/674,612, the entire contents of which are incorporated herein by reference. Alternatively or in addition, gels may be injected between tissues, especially after surgery, to prevent adhesion. Gels injected into open wounds can help prevent adhesion of dressings to the underlying tissues. Alternatively, gels may be injected under the abdominal periosteum to prevent the formation of adhesions after abdominal surgery.

Gels may also be injected into heart muscle to stimulate muscle production at thinning cardiac walls, as discussed in U.S. Patent Publication No. 2004- 0242469, the contents of which are incorporated herein by reference. Without being bound by a particular theory, we believe that peptide gel injected into certain tissue sites creates a permissive cavity for cell ingrowth and tissue development. The pH of the gel may be adjusted to further promote cell ingrowth and extracellular matrix production, or growth factors may be added to the peptide gel to promote specific cell behavior.

The techniques of the invention may also be used to heal orthopedic defects. For example, gels produced using the techniques described herein may be disposed around dental implants to bridge any gap between the implant and surrounding tissue and to promote ingrowth into implants. Gels may be injected into cartilage defects or osteo-chondral defects to prevent scar formation. Gels may also be used to coat the internal surfaces or fill pores in ceramic scaffolds. For example, a three-dimensional block of calcium phosphate or hydroxyapatite may be infused with a peptide solution before or after gelation. Infusion may be assisted by pressurizing the peptide solution or drawing a vacuum to promote infiltration. Alternatively or in addition, ceramic particles, especially crystalline, semi-crystalline, or amorphous calcium phosphate materials, may be combined with a peptide solution to form an injectable composition. Figure 2 shows photomicrographs of calvarial bone defects filled with phosphate-buffered (PBS) 1% and 3% solutions of peptide chains produced according to an embodiment of the invention. Both peptide concentrations facilitated bone healing superior to COLLAGRAFT^{™}, a collagen/tricalcium phosphate product, or COLLAPLUG^{™}, an absorbable collagen product. The peptide solution facilitated development of bone tissue, with few or no gaps in the defect site, while defects filled with COLLAGRAFT^{™} developed fibrous scar tissue, as did an untreated control defect. Better results were achieved with higher concentrations, combinations of the peptide gel with tricalcium phosphate, and assembly of the gel using an electolyte. The untreated control exhibited very little healing and minimal new bone (Figure 2B). The 3% unassembled peptide chain solution resulted in a gap about half the size of the empty control, with more osteoid, good vascularization, and no inflammatory reaction (Figure 2C). Treatment with Collaplug resulted in discontinuous islands of bone and formation of fibrous scar (Figure 2D). Treatment with an 3% peptide chain solution assembled in NaCl resulted in continuous, thicker bone (Figure 2E), while treatment with the same solution assembled in media resulted in discontinuous bone and the formation of fibrous tissue (Figure 2F). When an unassembled 3% solution combined with blood was employed, the defect site was detectable but much smaller than the original defect (Figure 2G). Combination of Collagraft and blood resulted in the development of fibrous tissue and very thin bone. The implant resorbed very slowly (Figure 2H). A 3% solution of peptide chains assembled in media and combined with tricalcium phosphate facilitated continuous, complete healing of the defect site (Figure 21). A 1:1 mixture of TCP and blood provided discontinuous healing; the fragments were surrounded by osteoblasts but resorbed slowly (Figure 2J). A 1% solution of peptide chains assembled in media was runny and not ideal for implantation or combination with other materials; however, it did facilitate the development of continuous bone in the defect site, although the bone was not as thick as after treatment with higher concentration solutions (Figure 2K)

The gels described herein also find utility in opthalmic applications. For example, gels may be used for short or long term repair of retinal detachment. The gel is injected into the eyeball, where the additional pressure presses the retina against the wall of the eye. Because the gel is transparent, lasers may still be used to afterwards permanently fix the retina in place. In prior art therapeutic techniques, patients often have to maintain their heads at awkward positions to retain an air bubble in place against the retina. The gels of the invention exert a constant hydrostatic pressure. As a result, we expect that, in applications where prior art therapies require an air bubble to be disposed against a particular point in the retina, replacing the air bubble with an incompressible gel may relieve at least some of the difficulty of convalescence after retinal surgery. Alternatively or in addition, peptide gels may be used for scleral buckling procedures. The gel is disposed against the outer surface of the eye to push the sclera towards the middle of the eye.

In any of the applications described above, peptide solutions may be injected before gelation. Indeed, peptide solutions may be injected before gelation in any application where ions will be able to migrate into the peptide solution from the surrounding tissue and cause it to gel. For example, where a long or narrow gauge needle is required or where it is advantageous for the gel to infiltrate dense tissue at the edges of a wound site, pre-gelation injection provides a more fluid material that will generate less back pressure during injection and can penetrate into dense fibrous tissues and inbetween mineralized bone fibrils. Rather than flowing away from the wound side, the fluid undergoes some self-assembly even before exposure to an electrolyte. We have observed spontaneous self-assembly of peptide chains in solutions with peptide chain concentrations of as great as 5%. This retains the material in place without the need for a cover or tissue flap. Of course, where the ionic strength of fluid at the injection site is insufficient, the peptide solution may be injected after gelation or may be followed by an electrolyte solution.

Optionally, one or more biologically active agents for example, therapeutically active compounds or chemoattractants, may be added to the peptide gels. Examples of such compounds include synthetic organic molecules, naturally occurring organic molecules, nucleic acid molecules, biosynthetic proteins such as chemokines, and modified naturally occurring proteins. Growth factors are also envisioned for use in this embodiment of the invention, alone or in combination with other biologically active agents. Exemplary growth factors include but are not limited to cytokines, epidermal growth factor, nerve growth factor, transforming growth factor-alpha and beta, platelet-derived growth factor, insulin-like growth factor, vascular endothelial growth factor, hematopoietic growth factor, heparin-binding growth factor, acidic fibroblast growth factor, basic fibroblast growth factor, hepatocyte growth factor, brain-derived neurotrophic factor, keratinocyte growth factor, bone morphogenetic protein, or a cartilage-derived growth factor.

For example, biologically active agents may also be added to the gels to recruit cells to a wound site or to promote production of extracellular matrix or the development of vascularization. Gels may also include compounds selected to reduce inflammation after implantation or to manage pain. Because the gels described herein degrade relatively quickly, analgesics, e.g., may be added to them without fear that pain killers will be continuously delivered at a wound site for months. In addition, powerful painkillers may be delivered locally to a wound site without having to deliver them systematically. Systemic delivery renders patients lethargic, and long term administration of opiates increases the risk of future drug dependency. Local administration leaves patients alert and can be continued for much longer periods of time.

Because the gel is injectable, it may be used for repeated, localized drug delivery. For example, it may be used to deliver anti-cancer drugs to a patient for long term therapy. Traditional chemotherapy techniques distribute toxic drugs throughout a patient's body. The techniques of the invention may be used to deliver chemotherapeutic agents to a specific site and release them quickly or over a period of days or weeks. Instead of repeated systemic administration, gels containing the desired agent may be periodically delivered directly to a desired site. Alternatively or in addition, anti-cancer agents may be locally delivered to a site from which a tumor was just removed. Peptide gels may be used as drug depots to store drugs and release them over long periods of time. Even biologicals such as proteins are stabilized by the peptide scaffold and may be released over a period of days, weeks, or months without losing their potency.

The peptide gels may be produced with encapsulated biologically active agents and stored for extended periods of time. That is, it is not necessary to store the added agent and the peptide separately and to mix them before delivery. Where the agent itself is ionic or is commonly stored in ionic media, addition of the biologically active agent to a peptide solution will cause the solution to gel, trapping the agent. Alternatively, the agent may be combined with an ionic solution and added to a peptide solution. We have discovered that antibodies added to an un-gelled peptide solution both gel the peptide solution and remain stable, without diffusing out of the gel, for at least nine weeks. The extended shelf life of a gel-drug mixture increases convenience for the health care provider and reduces the risk of contamination and infection from mixing the materials together or transferring material from one vial to another. In some cases, it may be possible to train patients to inject themselves. Where repeated administration over an extended time period is indicated, self-administration can help the patient reduce hospital visits. In an alternative embodiment, the peptide solution and the added agent are combined without added electrolyte. After injection, ions migrate into the solution from the surrounding tissue to gel the peptide solution and encapsulate the agent.

In another embodiment, biologically active agents may be tethered directly to the peptide chains. For example, such agents may be tethered to the ends of peptide chains during peptide synthesis. Alternatively or in addition, they may be linked via aggrecan processing sites such as those described in connection with Table 3. While there are agents that are sufficiently large to interfere with self-assembly of the peptide chains, these are not necessarily unsuitable for use with the compositions of the invention. Rather, the agent will simply form a bulge in the beta-sheet structure of the assembled peptide chains.

The concentration or composition of materials that are combined with the gels described herein may be varied. For example, a gel capsule may be produced containing a particular molecule at a given concentration. That capsule may itself be encapsulated within a larger capsule or in a matrix containing a number of like capsules. The larger capsule or matrix may contain a different molecule, for example, an additional biologically active agent, or a different concentration of the same material.

Cells may also be encapsulated within the scaffolds, as described, e.g., in copending applications U.S.S.N. 09/778,200, entitled "Peptide Scaffold Encapsulation of Tissue Cells and Uses Thereof", filed February 6, 2001, and in U.S.S.N. 10/877,068, entitled "Self-Assembling Peptides Incorporating Modifications", filed June 25, 2004, both of which are incorporated herein by reference. In case of conflict between the instant specification and the incorporated references, the instant specification shall control.

In another embodiment, the purified peptide product may be provided as part of a kit. The kit may include a purified peptide composition either in dry form or in a solution, and one or more of an electrolyte, a buffer, a delivery device, a vessel suitable for mixing the peptide composition with one or more other agents, instructions for preparing the peptide composition for use, instructions for mixing the peptide composition with other agents, and instructions for introducing the peptide composition into a subject. The delivery device may be, for example, a catheter, a needle, a syringe, or a combination of any of these. Where the kits are provided with an aqueous solution of peptide chains, the solution may have a shelf life of at least nine weeks under predetermined conditions and may include an electrolyte.

Such kits may deliver a biologically active agent in addition to the purified peptide composition. The biologically active agent may be provided pre-mixed with the peptide composition or separately. The biologically active agent may be present in nanospheres, microspheres, etc. (also referred to as nanocapsules, microcapsules, etc.). Numerous methods and reagents for making such spheres, capsules, etc., encapsulating a biologically active agent are known in the art. For example, standard polymers and methods for making sustained release and/or pH-resistant drug formulations can be used.

Similarly, the kits may be used to deliver cells to a patient. The kit may further include instructions for the preparation of cells to be delivered using the kit. For example, the instructions may describe how to culture cells, how to harvest cells from a subject, how to mix cells with the peptides, types of cells suitable for use, etc.

Other embodiments of the invention will be apparent to those skilled in the art from a consideration of the specification or practice of the invention disclosed herein. It is intended that the specification and examples be considered as exemplary only, with the true scope and spirit of the invention being indicated by the following claims.

Certain embodiments of the invention are set out in the following numbered paragraphs:
1. A composition, comprising: a plurality of amphiphilic peptide chains each having alternating hydrophilic and hydrophobic amino acids, wherein the peptide chains each contain at least 8 amino acids, are complementary and structurally compatible, and self-assemble into a beta-sheet macroscopic scaffold, and wherein at least about 75% of the peptide chains have the same sequence.
2. The composition of paragraph 1, wherein at least about 80% of the peptide chains have the same sequence.
3. The composition of paragraph 1, wherein at least about 85% of the peptide chains have the same sequence.
4. The composition of paragraph 1, wherein at least about 90% of the peptide chains have the same sequence.
5. The composition of paragraph 1, wherein at least about 95% of the peptide chains have the same sequence.
6. The composition of paragraph 1, wherein at least about 99% of the peptide chains have the same sequence.
7. The composition of paragraph 1, wherein the peptide has a sequence selected from 
RADARADARADARADA, RADARGDARADARGDA,
RARADADARARADADA, AEAKAEAKAEAKAEAK,
RAEARAEARAEARAEA, KADAKADAKADAKADA,
AEAEAHAHAEAEAHAH, FEFEFKFKFEFEFKFK, FKFEFKFEFKFEFKFE,
LELELKLKLELELKLK, AEAEAKAKAEAEAKAK,
KAKAKAKAEAEAEAEA, AEAEAEAEAKAKAKAK,
RARARARADADADADA, ADADADADARARARAR,
DADADADARARARARA, and HEHEHKHKHEHEHKHK.
8. The composition of paragraph 1, wherein the peptide has a sequence selected from
   AEAKAEAEAKAK, KLDLKLDLKLDL, FKFEFKFEFKFE,
   FKFQFKFQFKFQ, IKIEIKIEIKIE, and VKVEVKVEVKVE.
9. The composition of paragraph 1, wherein the peptide has a sequence selected from
   RADARADA, RARADADA, AEAKAEAK, KADAKADA, AEAEAHAH,
   FEFKFEFK, LELELKLK, AEAEAKAK, HEHEHKHK, RAEARAEA, and
   FKFEFKFE.
10. The composition of paragraph 1,, wherein the peptide has a sequence selected from
   VEVEVEVEVEVEVEVEVEVE and RFRFRFRFRFRFRFRFRFRF.
11. The composition of paragraph 1, wherein the peptide sequence is
   ADADADADARARARARADADADADARARARAR.
12. An aqueous solution comprising the composition of paragraph 1.
13. The aqueous solution of paragraph 12, wherein the solution forms a hydrogel that is stable with respect to mechanical agitation.
14. The aqueous solution of paragraph 12, wherein the solution is injectable.
15. The aqueous solution of paragraph 12, wherein the pH of the aqueous solution is between about 4.5 and about 8.5.
16. The aqueous solution of paragraph 12, wherein the peptides are self-assembled into a matrix in the solution.
17. The aqueous solution of paragraph 16, wherein the self-assembly is not stable with respect to mechanical agitation.
18. The aqueous solution of paragraph 16, further comprising at least about 0.1mM of an electrolyte.
19. The aqueous solution of paragraph 18, wherein the solution is injectable.
20. The aqueous solution of paragraph 14 or 19, wherein the peptide chains are capable of self-assembly after injection.
21. The aqueous solution of paragraph 12, further comprising a buffer.
22. The aqueous solution of paragraph 12, wherein the concentration of the peptide chains in the aqueous solution is at least about 1% by weight.
23. The aqueous solution of paragraph 12, wherein the concentration of the peptide chains in the aqueous solution is at least about 2% by weight.
24. The aqueous solution of paragraph 12, wherein the concentration of the peptide chains in the aqueous solution is at least about 3% by weight.
25. The aqueous solution of paragraph 12, wherein the concentration of the peptide chains in the aqueous solution is at least about 4% by weight.
26. The aqueous solution of paragraph 12, wherein the concentration of the peptide chains in the aqueous solution is at least about 5% by weight.
27. The aqueous solution of paragraph 12, wherein the concentration of the peptide chains in the aqueous solution is at least about 6% by weight.
28. The aqueous solution of paragraph 12, wherein the concentration of the peptide chains in the aqueous solution is at least about 7% by weight.
29. The aqueous solution of paragraph 12, wherein the concentration of the peptide chains in the aqueous solution is at least about 8% by weight.
30. The aqueous solution of paragraph 1 or 12, further comprising one or more biologically active agents.
31. A method of preparing a peptide chain, comprising: providing amino acids comprising a group in the side chain that is linked to a protecting group; assembling the amino acids into an amphiphilic peptide chain; removing the protecting group by reacting the peptide chain with an acid; and exchanging the acid for an non-fluorinated acetate salt.
32. The method of paragraph 31, wherein the non-fluorinated acetate salt is selected from sodium acetate and potassium acetate.
33. The method of paragraph 31, wherein the method further comprises preparing a plurality of peptide chains, at least about 75% of which have the same sequence.
34. The method of paragraph 31, wherein the method further comprises preparing a plurality of peptide chains, at least about 80% of which have the same sequence.
35. The method of paragraph 31, wherein the method further comprises preparing a plurality of peptide chains, at least about 85% of which have the same sequence.
36. The method of paragraph 31, wherein the method further comprises preparing a plurality of peptide chains, at least about 90% of which have the same sequence.
37. The method of paragraph 31, wherein the method further comprises preparing a plurality of peptide chains, at least about 95% of which have the same sequence.
38. The method of paragraph 31, wherein the method further comprises preparing a plurality of peptide chains, at least about 99% of which have the same sequence.
39. The method of paragraph 31, wherein the acid is trifluoroacetic acid.
40. A composition, comprising: an aqueous solution greater than 2% amphiphilic peptide chains having alternating hydrophilic and hydrophobic amino acids, wherein the peptide chains each contain at least 8 amino acids.
41. The composition of paragraph 40, wherein the peptide chains form a hydrogel that is stable with respect to mechanical agitation.
42. The composition of paragraph 40, wherein the solution is injectable.
43. The composition of paragraph 40, wherein the peptide chains are self-assembled into a matrix in the solution.
44. The composition of paragraph 43, wherein the self-assembly is not stable with respect to mechanical agitation.
45. The composition of paragraph 43, wherein the solution contains at least about 0.1mM of an electrolyte.
46. The composition of paragraph 45, wherein the solution is injectable.
47. The composition of paragraph 42 or 46, wherein the peptide chains are capable of self-assembly after injection.
48. The composition of paragraph 40, further comprising one or more biologically active agents.
49. The composition of paragraph 40, wherein the concentration of the peptide chains in the aqueous solution is at least about 3% by weight.
50. The composition of paragraph 40, wherein the concentration of the peptide chains in the aqueous solution is at least about 4% by weight.
51. The composition of paragraph 40, wherein the concentration of the peptide chains in the aqueous solution is at least about 5% by weight.
52. The composition of paragraph 40, wherein the concentration of the peptide in chains the aqueous solution is at least about 6% by weight.
53. The composition of paragraph 40, wherein the concentration of the peptide in chains the aqueous solution is at least about 7% by weight.
54. The composition of paragraph 40, wherein the concentration of the peptide chains in the aqueous solution is at least about 8% by weight.
55. A macroscopic scaffold comprising amphiphilic peptide chains, wherein the peptide chains have alternating hydrophobic and hydrophilic amino acids, are complementary and structurally compatible, and self-assemble into a beta-sheet macroscopic scaffold and wherein the amphiphilic peptide chains are in an aqueous solution, and wherein at least about 75% of the peptide chains have the same sequence.
56. The macroscopic scaffold of paragraph 55, wherein at least about 80% of the peptide chains have the same sequence.
57. The macroscopic scaffold of paragraph 55, wherein at least about 85% of the peptide chains have the same sequence.
58. The macroscopic scaffold of paragraph 55, wherein at least 90% of the peptide chains have the same sequence.
59. The macroscopic scaffold of paragraph 55, wherein at least 95% of the peptide chains have the same sequence.
60. The macroscopic scaffold of paragraph 55, wherein at least about 99% of the peptide chains have the same sequence.
61. The macroscopic scaffold of paragraph 55, wherein the concentration of the peptide chains in the aqueous solution is at least about 1% by weight.
62. The macroscopic scaffold of paragraph 55, wherein the concentration of the peptide chains in the aqueous solution is at least about 2% by weight.
63. The macroscopic scaffold of paragraph 55, wherein the concentration of the peptide chains in the aqueous solution is at least about 3% by weight.
64. The macroscopic scaffold of paragraph 55, wherein the concentration of the peptide chains in the aqueous solution is at least about 4% by weight.
65. The macroscopic scaffold of paragraph 55, wherein the concentration of the peptide chains in the aqueous solution is at least about 5% by weight.
66. The macroscopic scaffold of paragraph 55, wherein the concentration of the peptide chains in the aqueous solution is at least about 6% by weight.
67. The macroscopic scaffold of paragraph 55, wherein the concentration of the peptide chains in the aqueous solution is at least about 7% by weight.
68. The macroscopic scaffold of paragraph 55, wherein the concentration of the peptide chains in the aqueous solution is at least about 8% by weight.
69. The macroscopic scaffold of paragraph 55, wherein the scaffold is stable with respect to physical agitation.
70. The macroscopic scaffold of paragraph 69, wherein the aqueous solution containing the amphiphilic peptides is injectable.
71. The macroscopic scaffold of paragraph 55, wherein the scaffold is not stable with respect to physical agitation.
72. The macroscopic scaffold of paragraph 70, wherein the peptide chains are capable of self-assembly after injection.
73. The macroscopic scaffold of paragraph 55, wherein the amphiphilic peptides are in an aqueous solution containing an electrolyte, and wherein the scaffold is stable with respect to physical agitation.
74. The macroscopic scaffold of paragraph 73, wherein the aqueous solution containing the amphiphilic peptide chains is injectable.
75. The macroscopic scaffold of paragraph 55, further comprising a biologically active agent encapsulated within the scaffold.
76. A method of delivering a biologically active agent to a patient, comprising: providing an aqueous solution according to paragraph 12; adding a predetermined amount of the biologically active agent to the aqueous solution; and adding a predetermined amount of an electrolyte to the aqueous solution, wherein, after adding the predetermined amounts, the aqueous solution forms a hydrogel.
77. The method of paragraph 76, wherein the biologically active agent and the electrolyte are combined in a single aqueous solution and are added to the aqueous solution of the amphiphilic peptide simultaneously.
78. The method of paragraph 76, wherein the biologically active agent is selected from an anti-inflammatory, an antibiotic, an anti-cancer agent, an analgesic, an opioid, a drug, a growth factor, a protein, an amino acid, a peptide, a polynucleotide, a nucleotide, a carbohydrate, a sugar, a lipid, a polysaccharide, a nucleoprotein, a glycoprotein, a lipoprotein, a steroid, a chemoattractant, and any combination of the above.
79. The method of paragraph 76, further comprising injecting the hydrogel into a predetermined site in a patient.
80. The method of paragraph 76, further comprising injecting the aqueous solution into a patient before adding a predetermined amount of electrolyte and after adding a predetermined amount of said biologically active agent, wherein adding a predetermined amount of electrolyte comprises allowing ions to migrate into the injected solution from surrounding tissue.
81. The method of paragraph 76, further comprising injecting the aqueous solution into a predetermined site in a patient before adding a predetermined amount of electrolyte and after adding a predetermined amount of said biologically active agent, wherein the injected aqueous solution is stable with respect to migration from the predetermined site.
82. A kit for delivering a material to a patient, comprising: the composition of paragraph 1; and one or more of an electrolyte, a buffer, a delivery device, a vessel suitable for mixing the composition with one or more other agents, instructions for preparing the composition for use, instructions for mixing the composition with other agents, and instructions for introducing the composition into a subject.
83. The kit of paragraph 82, wherein the composition is in dry form or in an aqueous solution.
84. The kit of paragraph 83, wherein the aqueous solution has a shelf life of at least nine weeks under predetermined conditions.
85. The kit of paragraph 83, wherein the aqueous solution further comprises an electrolyte.
86. The kit of paragraph 82, wherein the delivery device comprises one or more of a catheter, a needle, and a syringe.
87. The kit of paragraph 82, further comprising a biologically active agent.
88. The kit of paragraph 87, wherein the biologically active agent is pre-mixed with the composition.
89. The kit of paragraph 87, wherein the biologically active agent is in the form of a nanosphere or a microsphere.
90. The kit of paragraph 82, further comprising instructions for the preparation of cells to be delivered using the kit.

## Claims

1. A method of preparing a composition comprising a plurality of amphiphilic peptide chains each having alternating hydrophilic and hydrophobic amino acids, wherein the peptide chains each contain at least 8 amino acids, are complementary and structurally compatible, and self-assemble into a beta-sheet macroscopic scaffold, and wherein at least about 75% of the peptide chains have the same sequence, and the peptide chains are at least 75% pure with respect to other chemical species, including deletion adducts and peptides of differing lengths, wherein the method comprises a purification step which separates the peptide chains having the desired sequence from: (a) excess reagents; and (b) deletion adducts, thereby increasing the solubility of the peptide chains in aqueous media.

2. The method of claim 1, comprising the steps of:
a. providing amino acids comprising a group in the side chain that is linked to a protecting group;
b. assembling the amino acids into an amphiphilic peptide chain;
c. removing the protecting group by reacting the peptide chain with an acid; and
d. exchanging the acid for a non-fluorinated acetate salt.

3. The method of claim 2, wherein the non-fluorinated acetate salt is selected from sodium acetate and potassium acetate.

4. The method of any one of the preceding claims, wherein at least about 80% of the peptide chains have the same sequence.

5. The method of any one of the preceding claims, wherein at least about 85% of the peptide chains have the same sequence.

6. The method of any one of the preceding claims, wherein at least about 90% of the peptide chains have the same sequence.

7. The method of any one of the preceding claims, wherein at least about 95% of the peptide chains have the same sequence.

8. The method of any one of the preceding claims, wherein at least about 99% of the peptide chains have the same sequence.

9. The method of any one of claims 2-8, wherein the acid is trifluoroacetic acid.

10. The method of claim 1, wherein the peptide chains are synthesised using solid phase techniques.

11. The method of claim 1, wherein the peptide chains are synthesised using solution phase techniques.

12. The method of claim 11, wherein the peptide is RAD16 having the sequence n-RADARADARADARADA-c.

13. The method of any one of the preceding claims, wherein the purification step comprises gel filtration and/or ion exchange high pressure liquid chromatography.

14. The method of any one of the preceding claims, wherein the purification step comprises repeated washing.


SEQUENCE LISTING

<110>  3D Matrix, Inc.

<120>  Purified Amphiphilic Peptide Compositions and Uses Thereof

<130>  2004837-0007

<140>  11/176,781
<141>  2005-07-06

<160>  50

<170>  PatentIn version 3.5

<210>  1
<211>  16
<212>  PRT
<213>  Artificial Sequence

<220>
<223>  Peptide sequences for cross-linking

<400>  1

Arg Gly Asp Tyr Arg Tyr Asp Tyr Arg Tyr Asp Tyr Arg Gly Asp Tyr
1               5                   10                  15


<210>  2
<211>  16
<212>  PRT
<213>  Artificial Sequence

<220>
<223>  Peptide sequences for cross-linking

<400>  2

Arg Gly Asp Phe Arg Phe Asp Phe Arg Phe Asp Phe Arg Gly Asp Phe
1               5                   10                  15


<210>  3
<211>  16
<212>  PRT
<213>  Artificial Sequence

<220>
<223>  Peptide sequences for cross-linking

<400>  3

Arg Gly Asp Trp Arg Trp Asp Trp Arg Trp Asp Trp Arg Gly Asp Trp
1               5                   10                  15


<210>  4
<211>  16
<212>  PRT
<213>  Artificial Sequence

<220>

<223>  Peptide sequences for cross-linking

<400>  4

Arg Ala Asp Tyr Arg Tyr Glu Tyr Arg Tyr Glu Tyr Arg Ala Asp Tyr
1               5                   10                  15


<210>  5
<211>  16
<212>  PRT
<213>  Artificial Sequence

<220>
<223>  Peptide sequences for cross-linking

<400>  5

Arg Ala Asp Phe Arg Phe Asp Phe Arg Phe Asp Phe Arg Ala Asp Phe
1               5                   10                  15


<210>  6
<211>  16
<212>  PRT
<213>  Artificial Sequence

<220>
<223>  Peptide sequences for cross-linking

<400>  6

Arg Ala Asp Trp Arg Trp Asp Trp Arg Trp Asp Trp Arg Ala Asp Trp
1               5                   10                  15


<210>  7
<211>  26
<212>  PRT
<213>  Artificial Sequence

<220>
<223>  Peptide sequences having aggrecan processing sites

<400>  7

Arg Gly Asp Tyr Arg Tyr Asp Tyr Thr Phe Arg Glu Glu Glu Gly Leu
1               5                   10                  15


Gly Ser Arg Tyr Asp Tyr Arg Gly Asp Tyr
            20                  25


<210>  8
<211>  26
<212>  PRT
<213>  Artificial Sequence

<220>
<223>  Peptide sequences having aggrecan processing sites

<400>  8

Arg Gly Asp Tyr Arg Tyr Asp Tyr Thr Phe Lys Glu Glu Glu Gly Leu
1               5                   10                  15

Gly Ser Arg Tyr Asp Tyr Arg Gly Asp Tyr
            20                  25

<210>  9
<211>  26
<212>  PRT
<213>  Artificial Sequence

<220>
<223>  Peptide sequences having aggrecan processing sites

<400>  9

Arg Gly Asp Tyr Arg Tyr Asp Tyr Thr Ala Ser Glu Leu Glu Glu Arg
1               5                   10                  15

Gly Thr Arg Tyr Asp Tyr Arg Gly Asp Tyr
            20                  25

<210>  10
<211>  27
<212>  PRT
<213>  Artificial Sequence

<220>
<223>  Peptide sequences having aggrecan processing sites

<400>  10

Arg Gly Asp Tyr Arg Tyr Asp Tyr Ala Pro Thr Ala Gly Glu Ala Gly
1               5                   10                  15

Glu Gly Pro Arg Tyr Asp Tyr Arg Gly Asp Tyr
            20                  25

<210>  11
<211>  27
<212>  PRT
<213>  Artificial Sequence

<220>
<223>  Peptide sequences having aggrecan processing sites

<400>  11

Arg Gly Asp Tyr Arg Tyr Asp Tyr Pro Thr Ile Ser Gln Glu Leu Gly
1               5                   10                  15

Gln Arg Pro Arg Tyr Asp Tyr Arg Gly Asp Tyr
            20                  25

<210>  12
<211>  27
<212>  PRT
<213>  Artificial Sequence

<220>
<223>  Peptide sequences having aggrecan processing site

<400>  12

Arg Gly Asp Tyr Arg Tyr Asp Tyr Pro Thr Val Ser Gln Glu Leu Gly
1               5                   10                  15

Gln Arg Pro Arg Tyr Asp Tyr Arg Gly Asp Tyr
            20                  25

<210>  13
<211>  16
<212>  PRT
<213>  Artificial Sequence

<220>
<223>  Self-assembling peptides

<400>  13

Arg Ala Asp Ala Arg Ala Asp Ala Arg Ala Asp Ala Arg Ala Asp Ala
1               5                   10                  15

<210>  14
<211>  16
<212>  PRT
<213>  Artificial Sequence

<220>
<223>  Self-assembling peptides

<400>  14

Arg Ala Asp Ala Arg Gly Asp Ala Arg Ala Asp Ala Arg Gly Asp Ala
1               5                   10                  15

<210>  15
<211>  8
<212>  PRT
<213>  Artificial Sequence

<220>
<223>  Self-assembling peptides

<400>  15

Arg Ala Asp Ala Arg Ala Asp Ala
1               5

<210>  16
<211>  16
<212>  PRT

<213>  Artificial Sequence

<220>
<223>  Self-assembling peptides

<400>  16

Arg Ala Arg Ala Asp Ala Asp Ala Arg Ala Arg Ala Asp Ala Asp Ala
1               5                   10                  15


<210>  17
<211>  8
<212>  PRT
<213>  Artificial Sequence

<220>
<223>  Self-assembling peptides

<400>  17

Arg Ala Arg Ala Asp Ala Asp Ala
1               5


<210>  18
<211>  16
<212>  PRT
<213>  Artificial Sequence

<220>
<223>  Self-assembling peptides

<400>  18

Ala Glu Ala Lys Ala Glu Ala Lys Ala Glu Ala Lys Ala Glu Ala Lys
1               5                   10                  15


<210>  19
<211>  8
<212>  PRT
<213>  Artificial Sequence

<220>
<223>  Self-assembling peptides

<400>  19

Ala Glu Ala Lys Ala Glu Ala Lys
1               5


<210>  20
<211>  16
<212>  PRT
<213>  Artificial Sequence

<220>
<223>  Self-assembling peptides

<400>  20

Arg Ala Glu Ala Arg Ala Glu Ala Arg Ala Glu Ala Arg Ala Glu Ala
1               5                   10                  15

<210>  21
<211>  8
<212>  PRT
<213>  Artificial Sequence

<220>
<223>  Self-assembling peptides

<400>  21

Arg Ala Glu Ala Arg Ala Glu Ala
1               5

<210>  22
<211>  16
<212>  PRT
<213>  Artificial Sequence

<220>
<223>  Self-assembling peptides

<400>  22

Lys Ala Asp Ala Lys Ala Asp Ala Lys Ala Asp Ala Lys Ala Asp Ala
1               5                   10                  15

<210>  23
<211>  8
<212>  PRT
<213>  Artificial Sequence

<220>
<223>  Self-assembling peptides

<400>  23

Lys Ala Asp Ala Lys Ala Asp Ala
1               5

<210>  24
<211>  12
<212>  PRT
<213>  Artificial Sequence

<220>
<223>  Self-assembling peptides

<400>  24

Lys Leu Asp Leu Lys Leu Asp Leu Lys Leu Asp Leu
1               5                   10

<210>  25
<211>  16
<212>  PRT

<213>  Artificial Sequence

<220>
<223>  Self-assembling peptides

<400>  25

Ala Glu Ala Glu Ala His Ala His Ala Glu Ala Glu Ala His Ala His
1               5                   10                  15


<210>  26
<211>  8
<212>  PRT
<213>  Artificial Sequence

<220>
<223>  Self-assembling peptides

<400>  26

Ala Glu Ala Glu Ala His Ala His
1               5


<210>  27
<211>  16
<212>  PRT
<213>  Artificial Sequence

<220>
<223>  Self-assembling peptides

<400>  27

Phe Glu Phe Glu Phe Lys Phe Lys Phe Glu Phe Glu Phe Lys Phe Lys
1               5                   10                  15


<210>  28
<211>  8
<212>  PRT
<213>  Artificial Sequence

<220>
<223>  Self-assembling peptides

<400>  28

Phe Glu Phe Lys Phe Glu Phe Lys
1               5


<210>  29
<211>  12
<212>  PRT
<213>  Artificial Sequence

<220>
<223>  Self-assembling peptides

<400>  29

Phe Lys Phe Glu Phe Lys Phe Glu Phe Lys Phe Glu
1               5                   10

<210>  30
<211>  8
<212>  PRT
<213>  Artificial Sequence

<220>
<223>  Self-assembling peptides

<400>  30

Phe Lys Phe Glu Phe Lys Phe Glu
1               5

<210>  31
<211>  16
<212>  PRT
<213>  Artificial Sequence

<220>
<223>  Self-assembling peptides

<400>  31

Phe Lys Phe Glu Phe Lys Phe Glu Phe Lys Phe Glu Phe Lys Phe Glu
1               5                   10                  15

<210>  32
<211>  12
<212>  PRT
<213>  Artificial Sequence

<220>
<223>  Self-assembling peptides

<400>  32

Phe Lys Phe Gln Phe Lys Phe Gln Phe Lys Phe Gln
1               5                   10

<210>  33
<211>  12
<212>  PRT
<213>  Artificial Sequence

<220>
<223>  Self-assembling peptides

<400>  33

Ile Lys Ile Glu Ile Lys Ile Glu Ile Lys Ile Glu
1               5                   10

<210>  34
<211>  12
<212>  PRT

<213>  Artificial Sequence

<220>
<223>  Self-assembling peptides

<400>  34

Val Lys Val Glu Val Lys Val Glu Val Lys Val Glu
1               5                   10


<210>  35
<211>  16
<212>  PRT
<213>  Artificial Sequence

<220>
<223>  Self-assembling peptides

<400>  35

Leu Glu Leu Glu Leu Lys Leu Lys Leu Glu Leu Glu Leu Lys Leu Lys
1               5                   10                  15


<210>  36
<211>  8
<212>  PRT
<213>  Artificial Sequence

<220>
<223>  Self-assembling peptides

<400>  36

Leu Glu Leu Glu Leu Lys Leu Lys
1               5


<210>  37
<211>  16
<212>  PRT
<213>  Artificial Sequence

<220>
<223>  Self-assembling peptides

<400>  37

Ala Glu Ala Glu Ala Lys Ala Lys Ala Glu Ala Glu Ala Lys Ala Lys
1               5                   10                  15


<210>  38
<211>  12
<212>  PRT
<213>  Artificial Sequence

<220>
<223>  Self-assembling peptides

<400>  38

Ala Glu Ala Glu Ala Glu Ala Glu Ala Lys Ala Lys
1               5                   10

<210>  39
<211>  8
<212>  PRT
<213>  Artificial Sequence

<220>
<223>  Self-assembling peptides

<400>  39

Ala Glu Ala Glu Ala Lys Ala Lys
1               5

<210>  40
<211>  16
<212>  PRT
<213>  Artificial Sequence

<220>
<223>  Self-assembling peptides

<400>  40

Lys Ala Lys Ala Lys Ala Lys Ala Glu Ala Glu Ala Glu Ala Glu Ala
1               5                   10                  15

<210>  41
<211>  16
<212>  PRT
<213>  Artificial Sequence

<220>
<223>  Self-assembling peptides

<400>  41

Ala Glu Ala Glu Ala Glu Ala Glu Ala Lys Ala Lys Ala Lys Ala Lys
1               5                   10                  15

<210>  42
<211>  16
<212>  PRT
<213>  Artificial Sequence

<220>
<223>  Self-assembling peptides

<400>  42

Arg Ala Arg Ala Arg Ala Arg Ala Asp Ala Asp Ala Asp Ala Asp Ala
1               5                   10                  15

<210>  43
<211>  16
<212>  PRT

<213>  Artificial Sequence

<220>
<223>  Self-assembling peptides

<400>  43

Ala Asp Ala Asp Ala Asp Ala Asp Ala Arg Ala Arg Ala Arg Ala Arg
1               5                   10                  15


<210>  44
<211>  16
<212>  PRT
<213>  Artificial Sequence

<220>
<223>  Self-assembling peptides

<400>  44

Asp Ala Asp Ala Asp Ala Asp Ala Arg Ala Arg Ala Arg Ala Arg Ala
1               5                   10                  15


<210>  45
<211>  16
<212>  PRT
<213>  Artificial Sequence

<220>
<223>  Self-assembling peptides

<400>  45

Ala Asp Ala Asp Ala Asp Ala Asp Ala Arg Ala Arg Ala Arg Ala Arg
1               5                   10                  15


<210>  46
<211>  16
<212>  PRT
<213>  Artificial Sequence

<220>
<223>  Self-assembling peptides

<400>  46

His Glu His Glu His Lys His Lys His Glu His Glu His Lys His Lys
1               5                   10                  15


<210>  47
<211>  8
<212>  PRT
<213>  Artificial Sequence

<220>
<223>  Self-assembling peptides

<400>  47

His Glu His Glu His Lys His Lys
1               5

<210>  48
<211>  20
<212>  PRT
<213>  Artificial Sequence

<220>
<223>  Self-assembling peptides

<400>  48

Val Glu Val Glu Val Glu Val Glu Val Glu Val Glu Val Glu Val Glu
1               5                   10                  15

Val Glu Val Glu
            20

<210>  49
<211>  20
<212>  PRT
<213>  Artificial Sequence

<220>
<223>  Self-assembling peptides

<400>  49

Arg Phe Arg Phe Arg Phe Arg Phe Arg Phe Arg Phe Arg Phe Arg Phe
1               5                   10                  15

Arg Phe Arg Phe
            20

<210>  50
<211>  5
<212>  PRT
<213>  Artificial Sequence

<220>
<223>  Self-assembling peptides

<400>  50

Arg Ala Asp Ala Arg
1               5